# EUROPÄISCHE PATENTSCHRIFT

(11) **EP 2 776 315 B1**
(45) Veröffentlichungstag und Bekanntmachung des Hinweises auf die Patenterteilung: **06.04.2016**
(21) Anmeldenummer: 13753588.6
(22) Anmeldetag: 19.07.2013
(51) Int. Cl.: B64C 29/00

(54) **SENKRECHT STARTENDES FLUGZEUG**
VERTICAL-TAKEOFF AIRCRAFT
AÉRONEF DÉCOLLANT À LA VERTICALE

(30) Priorität: 27.07.2012 DE 102012106869
(43) Veröffentlichungstag der Anmeldung: 17.09.2014
(73) Patentinhaber: Hesselbarth, Jonathan, 64283 Darmstadt (DE)
(72) Erfinder: Hesselbarth, Jonathan, 64283 Darmstadt (DE)
(74) Vertreter: Katscher Habermann Patentanwälte
(86) Internationale Anmeldenummer: PCT/EP2013/065361
(87) Internationale Veröffentlichungsnummer: WO 2014/016226

(56) Entgegenhaltungen:
- WO-A1-2012/141736
- GB-A- 2 376 928
- US-A- 3 089 666
- US-A- 3 231 221
- US-A- 3 469 803
- US-A- 4 492 353

## Beschreibung

Die Erfindung betrifft ein senkrecht startendes Flugzeug mit einem Flügel, wobei eine erste Antriebseinheit und eine zweite Antriebseinheit schwenkbar gelagert an dem Flügel angeordnet sind, wobei ein erster Abstand der ersten Antriebseinheit zu einer Längsachse des Flugzeugs näherungsweise gleich einem zweiten Abstand der zweiten Antriebseinheit zu der Längsachse des Flugzeugs ist und wobei die erste Antriebseinheit und die zweite Antriebseinheit in eine Horizontalfluglage und eine Vertikalfluglage schwenkbar sind, und wobei in der Vertikalfluglage die erste Antriebseinheit und die zweite Antriebseinheit in einer näherungsweise horizontalen Ebene angeordnet sind.

Ein solches Flugzeug ist aus US 3 231 221 A bekannt. Ein anderes senkrecht startendes Flugzeug ist aus GB 2 376 928 A bekannt.

Senkrecht startende Flugzeuge werden unter anderem als Drohnen und im militärischen Bereich eingesetzt. Diese Flugzeuge weisen üblicherweise zwei auf gegenüberliegenden Seiten eines Rumpfs angeordnete Flügel auf, wobei an den Flügeln jeweils zwei Antriebseinheiten in an den jeweiligen Einsatzzweck angepassten und mit den Flügeln starr verbundenen Tragelementen schwenkbar gelagert angeordnet sind. Es sind auch Flugzeuge bekannt, bei denen kein separater Rumpf ausgebildet ist und der Flügel aus zwei entlang der Längsache symmetrisch ausgestalteten Flügelhälften gebildet ist, wobei an den Flügelhälften jeweils zwei Antriebseinheiten in an den jeweiligen Einsatzzweck angepassten und mit den Flügelhälften starr verbundenen Tragelementen schwenkbar gelagert angeordnet sind.

Bei den Tragelementen kann es sich beispielsweise um unterhalb der Flügel angeordnete Gondeln handeln, in denen die Antriebseinheiten in Flugrichtung hintereinander schwenkbar gelagert angeordnet sind. Zum Starten und Landen bzw. im Schwebeflug werden die Antriebseinheiten in die Vertikalfluglage geschwenkt, wobei eine von den Antriebseinheiten bereitgestellte Antriebskraft im Wesentlichen vertikal in Richtung des Erdbodens wirkt und auf diese Weise eine vertikale Steig-, Sink- und Schwebefluglage ermöglicht. Sobald das Flugzeug eine Reiseflughöhe erreicht hat, werden die Antriebseinheiten in die Horizontalfluglage geschwenkt. In der Horizontalfluglage wirkt die von den Antriebseinheiten bereitgestellte Antriebskraft im Wesentlichen horizontal entgegen der Flugrichtung. Da bei senkrecht startenden Flugzeugen mit jeweils zwei in einer Gondel angeordneten Antriebseinheiten die Antriebseinheiten in der Gondel in Flugrichtung hintereinander angeordnet sind, strömt die in Flugrichtung vorne angeordnete Antriebseinheit die dahinter angeordnete Antriebseinheit an. Dadurch sinkt der Wirkungsgrad des senkrechtstartenden Flugzeugs in der Horizontalfluglage bezüglich einer zur Verfügung stehenden Rotorfläche.

Es ist auch bekannt, separate Tragelemente für die Antriebseinheiten an den Flügeln vorzusehen, wobei ein separates Tragelement oberhalb und ein separates Tragelement unterhalb des Flügels an dem Flügel starr angeordnet ist. Auf diese Weise strömen sich die Antriebseinheiten in der Horizontalfluglage nicht gegenseitig an, so dass der Wirkungsgrad in der Horizontalfluglage verbessert wird. Da die Antriebseinheiten in unterschiedlichen Höhen angeordnet sind, weisen die Antriebseinheiten im Vertikalflug in Bodennähe jedoch unterschiedliche Bodeneffekte auf, wobei ein von der tiefer angeordneten Antriebseinheit ausgehender Auftrieb üblicherweise größer ist, als ein von der höher angeordneten Antriebseinheit. Daher muss die Antriebsleistung der in unterschiedlichen Höhen angeordneten Antriebseinheiten im Vertikalflug ständig nachgeregelt werden. Dies führt zu einem unruhigen Flugverhalten im Vertikalflug.

Als Aufgabe der Erfindung wird es daher angesehen, die bekannten senkrecht startenden Flugzeuge so weiter zu entwickeln, dass im Horizontalflug ein möglichst guter Wirkungsgrad und im Vertikalflug ein möglichst ruhiges Flugverhalten erreicht wird.

Diese Aufgabe wird erfindungsgemäß dadurch gelöst, dass die gesamte Last der Antriebseinheiten über einen oder mehrere geeignete innerhalb des Flügels angeordnete Holme auf einen Rumpf des Flugzeugs übertragen wird, dass in der Horizontalfluglage die erste Antriebseinheit oberhalb einer Flügelfläche und die zweite Antriebseinheit unterhalb der Flügelfläche an dem Flügel angeordnet sind, und dass die erste Antreibseinheit und die zweite Antriebseinheit beabstandet zu einem einer Längsachse des Flugzeugs abgewandten Flügelende des Flügels an dem Flügel angeordnet sind.

Auf diese Weise erreicht man in der Vertikalflugphase in Bodennähe einen einheitlichen Bodeneffekt der ersten und zweiten Antriebseinheit, so dass ein ruhigeres Flugverhalten insbesondere in der Start- und Landephase erreicht wird. In der Horizontalfluglage strömen sich die erste Antriebseinheit die zweite Antriebseinheit nicht gegenseitig an, so dass hierdurch keine Wirkungsgradverluste eintreten. Die erste Antriebseinheit und die zweite Antriebseinheit sollten in der Vertikalfluglage möglichst in einer horizontalen Ebene angeordnet sein. Geringfügige Abweichungen von einer solchen Anordnung, die beispielsweise auf Fertigungstoleranzen zurückgeführt werden können, beeinträchtigen das Flugverhalten allerdings nicht oder lediglich geringfügig.

Vorteilhafterweise ist vorgesehen, dass die erste Antriebseinheit und die zweite Antriebseinheit jeweils einen Schwenkarm aufweisen, wobei die Schwenkarme schwenkbar gelagert an dem Flügel angeordnet sind. Auf diese Weise kann eine Schwenkachse der Antriebseinheiten so vorgegeben werden, dass die erste und die zweite Antriebseinheit in der Vertikalfluglage im Wesentlichen einer horizontalen Ebene angeordnet sind und in einer Horizontalfluglage oberhalb und unterhalb der Flügelfläche angeordnet sind. Zweckmäßigerweise sind die Schwenkarme so an dem Flügel angeordnet, dass sich die erste Antriebseinheit in der Vertikalfluglage in Horizontalflugrichtung vor dem Flügel und die zweite Antriebseinheit hinter dem Flügel befindet. Dabei können die Schwenkarme so ausgestaltet und an dem Flügel angeordnet sein, dass die erste Antriebseinheit und die zweite Antriebseinheit in der Horizontalfluglage im Wesentlichen in einer vertikalen Ebene unterhalb und oberhalb des Flügels angeordnet sind. Es ist aber auch möglich, dass die erste Antriebseinheit und die zweite Antriebseinheit in der Horizontalfluglage beabstandet zu einer vertikalen Ebene an dem Flügel angeordnet sind.

Das Flugverhalten des senkrecht startenden Flugzeugs kann dadurch weiter verbessert werden, dass ein Rumpfabstand der ersten Antriebseinheit und der zweiten Antriebseinheit zu der Längsachse des Flugzeugs kleiner ist als ein Flügelendeabstand der ersten Antriebseinheit und der zweiten Antriebseinheit zu einer durch das Flügelende verlaufenden und zu der Längsachse parallelen Flügelachse. Zweckmäßigerweise werden die Antriebseinheiten so nah wie möglich an dem Rumpf angeordnet. Ein kleinster möglicher Rumpfabstand ist beispielsweise durch einen Rotordurchmesser eines Rotors der ersten Antriebseinheit oder der zweiten Antriebseinheit begrenzt.

Erfindungsgemäss wird die gesamte Last der Antriebseinheiten über einen oder mehrere geeignete innerhalb des Flügels angeordnete Holme auf den Rumpf übertragen. Indem die Antriebseinheiten in der Nähe des Rumpfs an den Flügeln angeordnet werden, kann eine leichtere Flügelkonstruktion eingesetzt werden, da die Last der Antriebseinheiten nur durch einen kleinen Teil des Flügels zu dem Rumpf geleitet werden muss.

Aufgrund der Anordnung der Antriebseinheiten vor und hinter dem Flügel kann dieser verlängert und so zusätzliche Flügelstreckung generiert werden. Dadurch sinkt ein induzierter Widerstand der Flügel im Horizontalflug und die Flugleistungen werden verbessert.

Desweiteren wird durch die Anordnung der Antriebseinheiten in der Nähe des Rumpfs die Flügelkonstruktion insgesamt steifer, so dass geringere Biegeschwingungen in den Flügeln bzw. den tragenden Holmen auftreten. Dadurch wird insbesondere eine Manövrierbarkeit in der Vertikalfluglage verbessert und ein ruhigeres Flugverhalten erzielt.

Vorteilhafterweise ist erfindungsgemäß vorgesehen, dass an dem Flügel eine Schwenkvorrichtung angeordnet ist und die erste Antriebseinheit und die zweite Antriebseinheit mit der Schwenkvorrichtung in Wirkverbindung stehen. Durch die gemeinsame Nutzung der Schwenkvorrichtung für die erste und die zweite Antriebseinheit kann das Flügelgewicht weiter reduziert und das Flugverhalten des senkrecht startenden Flugzeugs weiter verbessert werden.

Um das senkrecht startende Flugzeug möglichst einfach regeln zu können und dadurch ein ruhigeres Flugverhalten zu ermöglichen ist erfindungsgemäß vorteilhafterweise vorgesehen, dass eine Schwenkbewegung der ersten Antriebseinheit und eine Schwenkbewegung der zweiten Antriebseinheit gekoppelt sind. Durch die Kopplung der Schwenkbewegung kann zudem auf komplexe Einzelantriebe zum unabhängigen verdrehen der Antriebseinheiten verzichtet werden und besonders kostengünstige und leichte Schwenkvorrichtungen eingesetzt werden.

Bei einer besonders vorteilhaften Ausgestaltung des erfindungsgemäßen senkrecht startenden Flugzeugs ist vorgesehen, dass die erste Antriebseinheit und die zweite Antriebseinheit eine Antriebsvorrichtung bilden und dass an dem Flügel mindestens zwei Antriebsvorrichtungen beabstandet zueinander angeordnet sind. Um eine Redundanz zu erhöhen kann demzufolge vorgesehen sein, dass zwei oder drei Antriebsvorrichtungen an dem Flügel angeordnet sind. Es können je nach Anforderung aber auch mehr als drei Antriebsvorrichtungen an dem Flügel angeordnet sein.

Eine besonders kostengünstige Konstruktion und ein besonders gutes Flugverhalten werden erfindungsgemäß dadurch erreicht, dass es sich bei der ersten Antriebseinheit und der zweiten Antriebseinheit um Propellerantriebe oder Impellerantriebe oder Strahltriebwerke handelt. Propellerantriebe sind zweckmäßigerweise starr oder mit einer Rotorblattverstellung ausgeführt.

Weitere Vorteile und Ausgestaltungen des senkrecht startenden Flugzeugs werden anhand der in der Zeichnung dargestellten Ausführungsbeispiele näher erläutert.

Es zeigt:
Figur 1 eine schematische Darstellung eines senkrecht startenden Flugzeugs mit seitlich an einem Flugzeugrumpf angeordneten Flügeln im Horizontalflug,
Figur 2 eine schematische Darstellung eines senkrecht startenden Flugzeugs mit seitlich an einem Flugzeugrumpf angeordneten Flügeln im Vertikalflug,
Figur 3 eine schematische Darstellung eines senkrecht startenden Flugzeugs ohne Heckleitwerk im Horizontalflug mit an einer Rumpfoberseite angeordneten Flügeln,
Figur 4a ein schematisch dargestellte Draufsicht auf ein senkrecht startendes Flugzeugs im Vertikalflug mit je zwei Antriebsvorrichtungen pro Flügel,
Figur 4b eine schematisch dargestellte Vorderansicht des in Figur 4a dargestellten senkrecht startenden Flugzeugs im Horizontalflug,
Figur 5 eine schematische Darstellung einer Schwenkvorrichtung mit an der Schwenkvorrichtung angeordneten Antriebseinheiten.

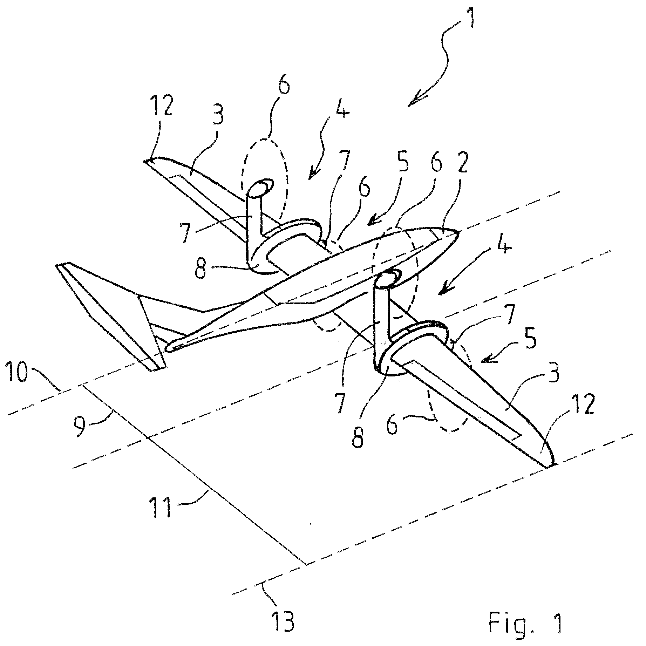

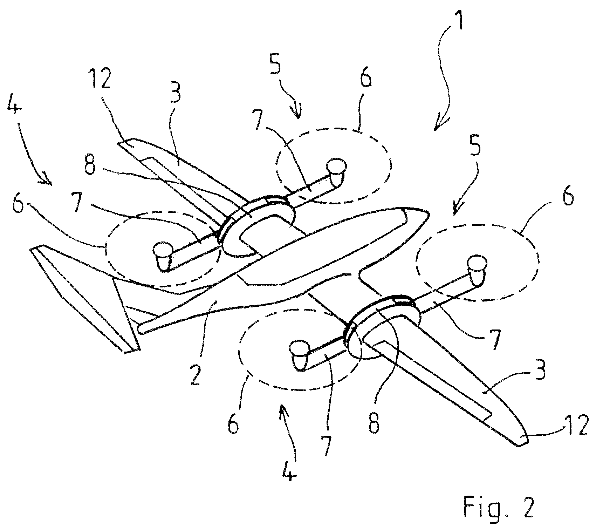

Figuren 1 und 2 zeigen schematische Darstellungen senkrecht startender Flugzeuge 1 mit je einem auf gegenüberliegenden Seiten eines Rumpfs 2 angeordneten Flügel 3. An den Flügeln 3 ist jeweils eine erste Antriebseinheit 4 und eine zweite Antriebseinheit 5 schwenkbar gelagert angeordnet. Die erste Antriebseinheit 4 und die zweite Antriebseinheit 5 weisen jeweils einen Propeller 6 und einen Schwenkarm 7 auf. Die Schwenkarme 7 sind schwenkbar an den Flügeln 3 angeordnet, wobei eine gekoppelte Schwenkbewegung von an den Flügeln 3 angeordneten Schwenkvorrichtungen 8 ausgelöst und angetrieben wird.

Die erste Antriebseinheit 4 und die zweite Antriebseinheit 5 sind so an den Flügeln 3 angeordnet, dass ein Rumpfabstand 9 der Antriebseinheiten 4 und 5 zu einer Längsachse 10 des senkrecht startenden Flugzeugs 1 kleiner ist als ein Flügelendeabstand 11 der ersten Antriebseinheit 4 und der zweiten Antriebseinheit 5 zu einer durch ein Flügelende 12 verlaufenden und zu der Längsachse 10 parallelen Flügelachse 13.

In Figur 1 befinden sich die erste Antriebseinheit 4 und die zweite Antriebseinheit 5 in der Horizontalfluglage während in Figur 2 die erste Antriebseinheit 4 und die zweite Antriebseinheit 5 in Vertikalfluglage dargestellt sind.

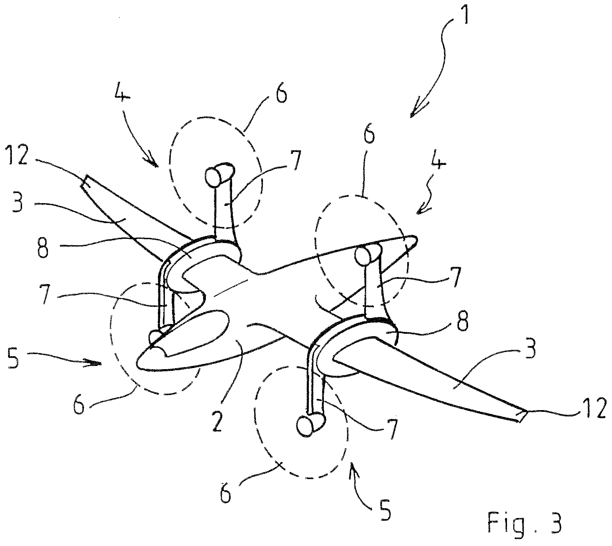

In Figur 3 ist schematisch ein senkrecht startendes Flugzeug 1 ohne Heckleitwerk mit zwei an einer Oberseite eines Rumpfs 2 angeordneten Flügeln 3 dargestellt.

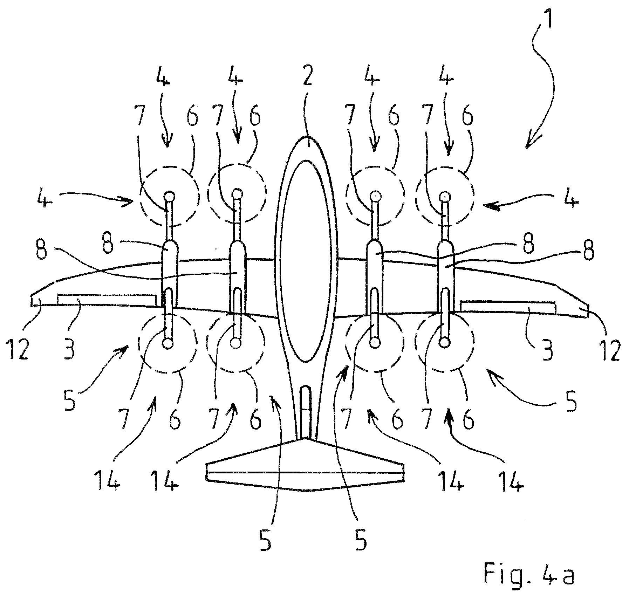

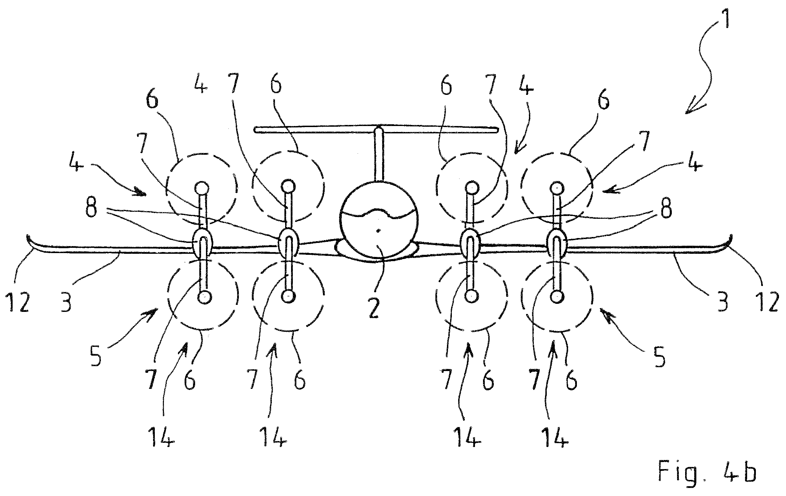

Figur 4a zeigt eine schematisch dargestellte Draufsicht und Figur 4b eine schematisch dargestellte Vorderansicht auf ein senkrecht startendes Flugzeug 1 mit zwei an gegenüberliegenden Seiten des Rumpfs 2 angeordneten Flügeln 3, wobei an den Flügeln 3 jeweils 2 Antriebsvorrichtungen 14 angeordnet sind. Die Antriebsvorrichtungen 14 weisen jeweils eine erste Antriebseinheit 4 und eine zweite Antriebseinheit 5 auf, die mit Schwenkarmen schwenkbar gelagert an den Flügeln 3 angeordnet sind.

Das senkrecht startende Flugzeug 1 ist in Figur 4a in der Vertikalflugphase dargestellt. In Figur 4b sind die Antriebseinheiten 4 und 5 in der Horizontalfluglage dargestellt.

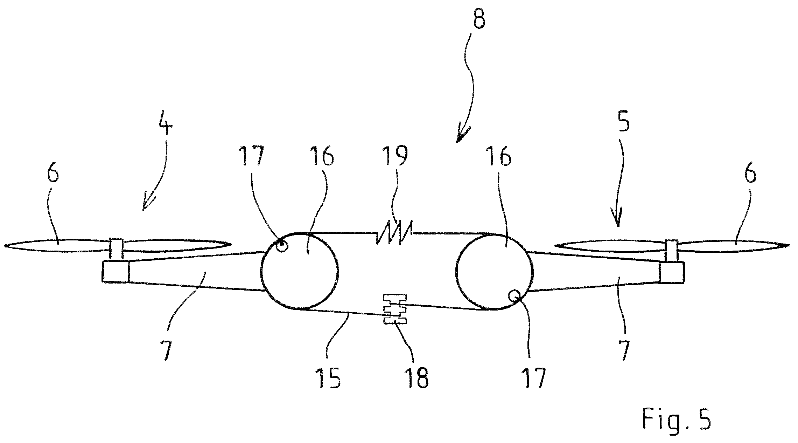

In Figur 5 ist schematisch eine Schwenkvorrichtung 8 mit zwei über einen Antriebsriemen 15 angetriebenen Antriebsrollen 16 dargestellt. Der Antriebsriemen 15 ist jeweils an Festlegepunkten 17 an den Antriebsrollen 16 festgelegt. Eine Lage des Antriebriemens 15 kann über eine Winde 18 verstellt werden. Zur Montage und Demontage des Antriebriemens 15 ist an dem Antriebsriemen 15 eine Spannvorrichtung 19 vorgesehen.

An den Antriebsrollen 16 sind eine erste Antriebseinheit 4 und eine zweite Antriebseinheit 5 angeordnet. Die erste Antriebseinheit 4 und die zweite Antriebseinheit 5 weisen jeweils einen Schwenkarm 7 und einen einem Schwenkarm 7 angeordneten Propeller 6 auf.

Durch verstellen der Lage des Antriebriemens 15 mithilfe der Winde 18 werden die Antriebsrollen 16 gekoppelt verdreht. Zum Antrieb der Winde 18 kann beispielsweise ein nicht dargestellter elektrischer Servoantrieb eingesetzt werden.

## Patentansprüche

1. Senkrecht startendes Flugzeug (1) mit einem Flügel (3), wobei eine erste Antriebseinheit (4) und eine zweite Antriebseinheit (5) schwenkbar gelagert an dem Flügel (3) angeordnet sind, wobei ein erster Abstand der ersten Antriebseinheit (4) zu einer Längsachse (10) des Flugzeugs (1) näherungsweise gleich einem zweiten Abstand der zweiten Antriebseinheit (5) zu der Längsachse (10) des Flugzeugs (1) ist, wobei die erste Antriebseinheit (4) und die zweite Antriebseinheit (5) in eine Horizontalfluglage und eine Vertikalfluglage schwenkbar sind, und wobei in der Vertikalfluglage die erste Antriebseinheit (4) und die zweite Antriebseinheit (5) in einer näherungsweise horizontalen Ebene angeordnet sind, **dadurch gekennzeichnet, dass** die gesamte Last der Antriebseinheiten (4, 5) über einen oder mehrere geeignete innerhalb des Flügels (3) angeordnete Holme auf einen Rumpf (2) des Flugzeugs (1) übertragen wird, dass in der Horizontalfluglage die erste Antriebseinheit (4) oberhalb einer Flügelfläche und die zweite Antriebseinheit (5) unterhalb der Flügelfläche an dem Flügel (3) angeordnet sind, und dass die erste Antriebseinheit (4) und die zweite Antriebseinheit (5) beabstandet zu einem einer Längsachse (10) des Flugzeugs (1) abgewandten Flügelende (12) des Flügels (3) an dem Flügel (3) angeordnet sind.

2. Senkrecht startendes Flugzeug (1) gemäß Anspruch 1, **dadurch gekennzeichnet, dass** die erste Antriebseinheit (4) und die zweite Antriebseinheit (5) jeweils einen Schwenkarm (7) aufweisen, wobei die Schwenkarme (7) schwenkbar gelagert an dem Flügel (3) angeordnet sind.

3. Senkrecht startendes Flugzeug (1) gemäß einem der voranstehenden Ansprüche, **dadurch gekennzeichnet, dass** ein Rumpfabstand (9) der ersten Antriebseinheit (4) und der zweiten Antriebseinheit (5) zu der Längsachse (10) des Flugzeugs (1) kleiner ist als ein Flügelendeabstand (11) der ersten Antriebseinheit (4) und der zweiten Antriebseinheit (6) zu einer durch das Flügelende (12) verlaufenden und zu der Längsachse (10) parallelen Flügelachse (13).

4. Senkrecht startendes Flugzeug (1) gemäß einem der voranstehenden Ansprüche, **dadurch gekennzeichnet, dass** an dem Flügel (3) eine Schwenkvorrichtung (8) angeordnet ist und die erste Antriebseinheit (4) und die zweite Antriebseinheit (5) mit der Schwenkvorrichtung (8) in Wirkverbindung stehen.

5. Senkrecht startendes Flugzeug (1) gemäß einem der voranstehenden Ansprüche, **dadurch gekennzeichnet, dass** eine Schwenkbewegung der ersten Antriebseinheit (4) und eine Schwenkbewegung der zweiten Antriebseinheit (5) gekoppelt sind.

6. Senkrecht startendes Flugzeug (1) gemäß einem der voranstehenden Ansprüche, **dadurch gekennzeichnet, dass** die erste Antriebseinheit (4) und die zweite Antriebseinheit (5) eine Antriebsvorrichtung (14) bilden und dass an dem Flügel (3) mindestens zwei Antriebsvorrichtungen (14) beabstandet zueinander angeordnet sind.

7. Senkrecht startendes Flugzeug (1) gemäß einem der voranstehenden Ansprüche, **dadurch gekennzeichnet, dass** es sich bei der ersten Antriebseinheit (4) und der zweiten Antriebseinheit (5) um Propellerantriebe (6) oder Impellerantriebe oder Strahltriebwerke handelt.

## Claims

1. Vertical-takeoff aircraft (1) with a wing (3), wherein a first drive unit (4) and a second drive unit (5) are arranged mounted pivotably on the wing (3), wherein a first distance of the first drive unit (4) from a longitudinal axis (10) of the aircraft (1) is approximately identical to a second distance of the second drive unit (5) from the longitudinal axis (10) of the aircraft (1), wherein the first drive unit (4) and the second drive unit (5) are pivotable into a horizontal flying position and a vertical flying position, and wherein, in the vertical flying position, the first drive unit (4) and the second drive unit (5) are arranged in an approximately horizontal plane, **characterized in that** the entire load of the drive units (4, 5) is transmitted to a fuselage (2) of the aircraft (1) by means of one or more suitable rails which are arranged within the wing (3), **in that**, in the horizontal flying position, the first drive unit (4) is arranged above a wing surface, and the second drive unit (5) is arranged below the wing surface, on the wing (3), and **in that** the first drive unit (4) and the second drive unit (5) are arranged on the wing (3) at a distance from an end (12) of the wing (3) facing away from a longitudinal axis (10) of the aircraft (1).

2. Vertical-takeoff aircraft (1) according to Claim 1, **characterized in that** the first drive unit (4) and the second drive unit (5) each have a pivoting arm (7), wherein the pivoting arms (7) are arranged mounted pivotably on the wing (3).

3. Vertical-takeoff aircraft (1) according to either of the preceding claims, **characterized in that** a fuselage distance (9) of the first drive unit (4) and of the second drive unit (5) from the longitudinal axis (10) of the aircraft (1) is smaller than a wing end distance (11) of the first drive unit (4) and of the second drive unit (6) from a wing axis (13) which runs through the wing end (12) and is parallel to the longitudinal axis (10).

4. Vertical-takeoff aircraft (1) according to one of the preceding claims, **characterized in that** a pivoting device (8) is arranged on the wing (3), and the first drive unit (4) and the second drive unit (5) are operatively connected to the pivoting device (8).

5. Vertical-takeoff aircraft (1) according to one of the preceding claims, **characterized in that** a pivoting movement of the first drive unit (4) and a pivoting movement of the second drive unit (5) are coupled.

6. Vertical-takeoff aircraft (1) according to one of the preceding claims, **characterized in that** the first drive unit (4) and the second drive unit (5) form a drive device (14), and **in that** at least two drive devices (14) are arranged spaced apart from each other on the wing (3).

7. Vertical-takeoff aircraft (1) according to one of the preceding claims, **characterized in that** the first drive unit (4) and the second drive unit (5) are propeller drives (6) or impeller drives or jet engines.

## Revendications

1. Aéronef décollant à la verticale (1), comprenant une aile (3), une première unité d'entraînement (4) et une deuxième unité d'entraînement (5) étant montées de manière pivotante sur l'aile (3), une première distance de la première unité d'entraînement (4) à un axe longitudinal (10) de l'aéronef (1) étant approximativement identique à une deuxième distance de la deuxième unité d'entraînement (5) à l'axe longitudinal (10) de l'aéronef (1), la première unité d'entraînement (4) et la deuxième unité d'entraînement (5) pouvant pivoter dans une position de vol horizontale et dans une position de vol verticale, et dans la position de vol verticale, la première unité d'entraînement (4) et la deuxième unité d'entraînement (5) étant disposées dans un plan approximativement horizontal, **caractérisé en ce que** la charge totale des unités d'entraînement (4, 5) est transmise par le biais d'un ou plusieurs longerons appropriés disposés à l'intérieur de l'aile (3) à un fuselage (2) de l'aéronef (1), **en ce que** dans la position de vol horizontale, la première unité d'entraînement (4) est disposée au-dessus d'une surface de l'aile et la deuxième unité d'entraînement (5) est disposée en dessous de la surface de l'aile au niveau de l'aile (3), et **en ce que** la première unité d'entraînement (4) et la deuxième unité d'entraînement (5) sont disposées au niveau de l'aile (3) à distance d'une pointe d'aile (12) de l'aile (3) opposée à un axe longitudinal (10) de l'aéronef (1).

2. Aéronef décollant à la verticale (1) selon la revendication 1, **caractérisé en ce que** la première unité d'entraînement (4) et la deuxième unité d'entraînement (5) présentent à chaque fois un bras pivotant (7), les bras pivotants (7) étant supportés de manière pivotante au niveau de l'aile (3).

3. Aéronef décollant à la verticale (1) selon l'une quelconque des revendications précédentes, **caractérisé en ce qu'**une distance au fuselage (9) de la première unité d'entraînement (4) et de la deuxième unité d'entraînement (5) à l'axe longitudinal (10) de l'aéronef (1) est inférieure à une distance de pointe d'aile (11) de la première unité d'entraînement (4) et de la deuxième unité d'entraînement (6) à un axe d'aile (13) parallèle à l'axe longitudinal (10) et s'étendant à travers la pointe d'aile (12).

4. Aéronef décollant à la verticale (1) selon l'une quelconque des revendications précédentes, **caractérisé en ce qu'**un dispositif de pivotement (8) est disposé au niveau de l'aile (3) et la première unité d'entraînement (4) et la deuxième unité d'entraînement (5) sont en liaison fonctionnelle avec le dispositif de pivotement (8).

5. Aéronef décollant à la verticale (1) selon l'une quelconque des revendications précédentes, **caractérisé en ce qu'**un mouvement de pivotement de la première unité d'entraînement (4) et un mouvement de pivotement de la deuxième unité d'entraînement (5) sont accouplés.

6. Aéronef décollant à la verticale (1) selon l'une quelconque des revendications précédentes, **caractérisé en ce que** la première unité d'entraînement (4) et la deuxième unité d'entraînement (5) forment un dispositif d'entraînement (14) et **en ce qu'**au moins deux dispositifs d'entraînement (14) sont disposés à distance l'un de l'autre au niveau de l'aile (3).

7. Aéronef décollant à la verticale (1) selon l'une quelconque des revendications précédentes, **caractérisé en ce que** la première unité d'entraînement (4) et la deuxième unité d'entraînement (5) sont des entraînements à hélice (6) ou des entraînements à turbine ou des moteurs à réaction.
